(19) Europäisches Patentamt
European Patent Office
Office européen des brevets

(11) **EP 2 773 265 B1**

(12) **EUROPÄISCHE PATENTSCHRIFT**

(45) Veröffentlichungstag und Bekanntmachung des Hinweises auf die Patenterteilung:
**30.12.2015 Patentblatt 2015/53**

(21) Anmeldenummer: **12778757.0**

(22) Anmeldetag: **31.10.2012**

(51) Int Cl.:
*A61B 5/145* (2006.01)   *G01N 33/487* (2006.01)

(86) Internationale Anmeldenummer:
**PCT/EP2012/071589**

(87) Internationale Veröffentlichungsnummer:
**WO 2013/064549 (10.05.2013 Gazette 2013/19)**

(54) **ANALYTISCHES HANDGERÄT UND VERFAHREN ZU DESSEN BETRIEB**

ANALYTICAL HAND-HELD DEVICE AND METHOD FOR ITS OPERATION

APPAREIL MANUEL ANALYTIQUE ET SON PROCÉDÉ DE FONCTIONNEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.11.2011 EP 11187840**

(43) Veröffentlichungstag der Anmeldung:
**10.09.2014 Patentblatt 2014/37**

(73) Patentinhaber:
• **Roche Diagnostics GmbH**
  **68305 Mannheim (DE)**
  Benannte Vertragsstaaten:
  **DE**
• **F.Hoffmann-La Roche AG**
  **4070 Basel (CH)**
  Benannte Vertragsstaaten:
  **AL AT BE BG CH CY CZ DK EE ES FI FR GB GR HR HU IE IS IT LI LT LU LV MC MK MT NL NO PL PT RO RS SE SI SK SM TR**

(72) Erfinder:
• **CHANG, Tso-Yu**
  **Shulin Dist. 238**
  **New Taipei City (TW)**
• **WANG, Peng-Kun**
  **Xiangshan Dist.,**
  **300 Hsinchu City (TW)**

(74) Vertreter: **Pfiz, Thomas et al**
**Wolf Pfiz & Gauss**
**Patentanwälte**
**Hauptmannsreute 93**
**70193 Stuttgart (DE)**

(56) Entgegenhaltungen:
EP-A1- 2 177 155      EP-A1- 2 279 689
US-A1- 2007 066 938   US-B1- 6 470 291

EP 2 773 265 B1

**Beschreibung**

Die Erfindung betrifft ein analytisches Handgerät, insbesondere für Blutzuckertests, mit einer austauschbaren Testbandkassette, die ein mit einer Vielzahl von analytischen Hilfsmitteln versehenes Testband enthält, einem einen Gleichstrommotor und ein mit der Testbandkassette koppelbares Getriebe aufweisenden Bandantrieb zum sukzessiven Bereitstellen der analytischen Hilfsmittel, und einer Regeleinrichtung zur Drehzahlregelung des Gleichstrommotors. Die Erfindung betrifft weiterhin ein Verfahren zum Betrieb eines solchen analytischen bzw. medizinischen Handgeräts.

Derartige Testbandsysteme wurden bereits in einer Reihe von Patentanmeldungen von den Anmeldern vorgeschlagen, um gegenüber den am Markt befindlichen Streifensystemen weitere Anwendervorteile zu gewinnen siehe z.B. EP 2 177 155 A1. Für die Praxis muss neben einer zuverlässigen Positionierung der Testelemente auch gewährleistet sein, dass eine vorgegebene Bereitstellungszeit für die Einzeltests mit möglichst geringem Aufwand eingehalten werden kann.

Ausgehend hiervon liegt der Erfindung die Aufgabe zugrunde, die im Stand der Technik vorgeschlagenen Systeme und Verfahren weiter zu verbessern und eine rasche Testelementbereitstellung bei vertretbarer Bandbelastung mit einfachen Mitteln zu erreichen.

Zur Lösung dieser Aufgabe wird die im Patentanspruch 1 bzw. 14 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, einen hochdrehenden Motor durch eine möglichst einfache Drehzahlregelung zu ergänzen. Dementsprechend wird erfindungsgemäß vorgeschlagen, dass die Regeleinrichtung einen an dem Gleichstrommotor angeordneten Drehgeber zur Erfassung der IST-Drehzahl der Motorabtriebswelle aufweist. Damit ist es möglich, kostengünstige kompakte Kleinmotoren einzusetzen und deren bauartbedingte Drehzahlvarianz zu kompensieren, ohne dass bereits bei der Herstellung eine Motorkalibrierung erforderlich wäre. Gleichzeitig kann die Motordrehzahl verbrauchsabhängig dahingehend kontrolliert werden, dass eine gleichbleibende Testbandgeschwindigkeit und damit eine konstante Testbereitstellungszeit erreicht wird. Die direkte Erfassung der Drehung der Motorabtriebswelle ermöglicht die erforderliche Genauigkeit der Regelung mit kurzer Totzeit, wobei die Erkenntnis zugrunde gelegt wird, dass durch die dem Motor nachgeordnete Antriebskopplung des Testbandes keine nennenswerten Störeinflüsse auftreten.

Um mit möglichst einfachen Mitteln eine hochpräzise Drehwinkelerfassung zu ermöglichen, ist es vorteilhaft, wenn der Drehgeber einen die Drehung der Motorabtriebswelle optisch abtastenden und dabei eine zur Drehzahl proportionale Rate von elektrischen Impulsen als Ausgangssignal erzeugenden opto-elektronischen Encoder aufweist.

Eine weitere Verbesserung lässt sich dadurch erzielen, dass der Drehgeber einen drehfest auf der Motorabtriebswelle sitzenden Unterbrecher, insbesondere ein Flügelrad oder eine Lochscheibe und eine mit dem Unterbrecher zusammenwirkende gerätefeste Lichtschranke, vorzugsweise eine Gabellichtschranke aufweist.

Zur möglichst präzisen Signalauswertung ist vorteilhafterweise ein zur Erzeugung von Zeittaktimpulsen ausgebildeter Taktgeber und ein Zähler zum Zählen von Zeittaktimpulsen zwischen zwei Signalflanken eines Ausgangssignals des Drehgebers vorgesehen.

Für eine möglichst variable Regelung ist es günstig, wenn die Regeleinrichtung einen Vergleicher zur Bildung einer Regeldifferenz zwischen der IST-Drehzahl und einer als Führungsgröße vorgegebenen SOLL-Drehzahl aufweist. Hierbei ist es auch von Vorteil, wenn die Regeleinrichtung einen eingangsseitig mit einer Regeldifferenz beaufschlagbaren Regelprozessor zur Einstellung einer Soll-Drehzahl in einem geschlossenen Regelkreis aufweist.

Für eine möglichst rasche Verringerung einer Regelabweichung sollte der Regelprozessor vorzugsweise durch eine Software-Routine gebildete Proportional- und Integral-Regelglieder aufweisen.

Zur genauen Stellgrößeneinstellung ist es vorteilhaft, wenn die Regeleinrichtung einen Pulsweitenmodular als Stellglied zur Ansteuerung des Gleichstrommotors mit einer pulsweitenmodulierten Gleichspannung aufweist.

Vorteilhafterweise ist die Regeleinrichtung dazu eingerichtet, eine Bandgeschwindigkeit des Testbandes von 15 +/- 2 mm/s mit einer Einstellzeit im Bereich von 0,1 bis 0,25 s zu erreichen.

Ein weiterer Gebrauchsvorteil für den Benutzer lässt sich dadurch erreichen, dass die Regeleinrichtung einen Sollwertgenerator zur Festlegung einer aktuellen SOLL-Drehzahl nach Maßgabe einer gleichbleibenden Bereitstellungszeit für die jeweilige Bereitstellung der analytischen Hilfsmittel aufweist.

In diesem Zusammenhang ist es auch vorteilhaft, einen Sollwertspeicher zur Speicherung einer Sollwerttabelle vorzusehen, in welcher einer Testnummer der fortlaufend nummerierten analytischen Hilfsmittel jeweils ein Wert für die SOLL-Drehzahl des Gleichstrommotors zugeordnet ist.

Um auch bei einem Kassettenwechsel eine Verbrauchsinformation bereitstellen zu können, ist es vorteilhaft, wenn die Testbandkassette mit einem insbesondere als RFID-Chip ausgebildeten Speichermittel zur verbrauchsabhängigen Speicherung einer Testnummer des aktuell bereitzustellenden analytischen Hilfsmittels versehen ist.

Der Testbandantrieb ist vorteilhafterweise dazu eingerichtet, die analytischen Hilfsmittel bei Bedarf durch Bandtransport von einer gegenüber der Umgebung abgeschirmten Vorratsspule abzuziehen und an einer Applikationsstelle eines Gerätegehäuses bereitzustellen.

In verfahrensmäßiger Hinsicht wird die eingangs genannte Aufgabe dadurch gelöst, dass die IST-Drehzahl der Motorabtriebswelle durch einen an dem Gleichstrommotor angeordneten Drehgeber der Regeleinrichtung erfasst wird.

Im Folgenden wird die Erfindung anhand eines in der Zeichnung dargestellten Ausführungsbeispiels näher erläutert. Es zeigen:

Fig. 1    ein analytisches Handgerät zum Einsatz einer wechselbaren Testbandkassette in einer perspektivischen Darstellung mit Detailvergrößerung eines Drehgebers;

Fig. 2    ein Blockschaltbild einer Regeleinrichtung für den Bandantrieb;

Fig. 3    ein Zeitdiagramm der Ausgangssignale des Drehgebers über den Taktimpulsen eines Zeittaktgebers.

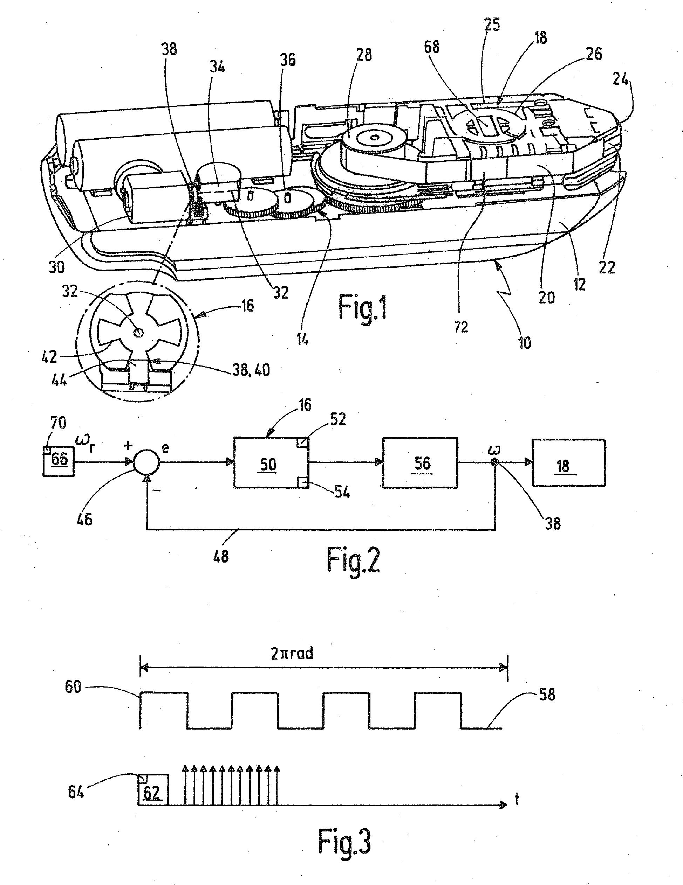

Das in Fig. 1 dargestellte Handgerät lässt sich von einem Anwender in der Hand tragen und dabei für rasche Blutzuckermessungen vor Ort einsetzen. Das Mobilgerät 10 umfasst zu diesem Zweck ein ohne Abdeckung gezeigtes Gehäuse 12 mit einem Bandantrieb 14 und einer darauf einwirkenden Regeleinrichtung 16 zur Bandtransportkontrolle einer als Verbrauchsmittel einwechselbaren Testbandkassette 18.

Die ohne Deckel, dargestellte Testbandkassette 18 enthält ein Testband 20, das abschnittsweise mit trockenchemischen Testfeldern 22 versehen ist, die im Bereich einer Umlenkspitze als Applikationsstelle 24 an ihrer freien Vorderseite mit Blut bzw. Körperflüssigkeit beaufschlagbar sind. Zugleich kann dort eine rückseitige Vermessung des momentan bzw. aktuell benutzten Testfelds 22 durch eine nicht gezeigte photometrische Messeinheit erfolgen. Das Testband 16 lasst sich hierzu mittels des Bandantriebs 14 aus einer abgedichteten Vorratskammer 25 von einer darin befindlichen Abwickelspule 26 auf eine Aufwickelspule 28 umspulen, so dass die voneinander beabstandeten Testfelder 22 nacheinander an der Applikationsstelle 24 für sukzessive Tests zum Einsatz gebracht werden können. Hierbei wird nur die Aufwickelspule 22 durch den über einen Mitnehmerzapfen angekoppelten Bandantrieb 14 angetrieben. Denkbar ist auch der Einsatz von Stechelementen oder integrierten Stech/Testelementen als analytische Hilfsmittel auf einem Testband.

Der Bandantrieb 14 weist einen als hochdrehender Gleichstrom-Kleinmotor ausgebildeten Motor 30 und ein nachgeordnetes Untersetzungsgetriebe auf, das ein auf der Motorabtriebswelle 32 sitzendes Schneckenrad 34 und ein mehrstufiges Stirnradgetriebe 36 umfasst. Bei einer Kreisfrequenz des Motors 30 im Bereich von ca. 600 bis 1000 rad/s lässt sich damit die Spulendrehzahl der Aufwickelspule 28 auf weniger als 1 Umdrehung in der Sekunde reduzieren, so dass in energieeffizienter und kompakter Bauweise das erforderliche Drehmoment bereitsteht, um auch den Banddurchzug durch die Abdichtung der Vorratskammer 25 hindurch zu gewährleisten.

Mit der Regeleinrichtung 16 lassen sich die für DC-Kleinmotoren bauartbedingten hohen Drehzahlvarianzen kompensieren, um eine übermäßige Zugbelastung des Testbandes 20 zu vermeiden und eine vorgegebene Bereitstellungszeit eines unverbrauchten Testfelds 22 beispielsweise im Bereich von 5 bis 10 s zu gewährleisten. Hierbei ist zu berücksichtigen, dass die auf dem unverbrauchten Abschnitt des Testbandes 20 in der Vorratskammer 25 befindlichen Testfelder 22 erst auf Anforderung des Benutzers jeweils einzeln ausgespult werden, um schädliche Umwelteinflüsse auf die empfindliche Testchemie zu vermeiden. Neben einem Ausgleich von Motordrehzahlvarianzen für eine konstante Testbereitstellungszeit lassen sich durch die Regeleinrichtung auch solche Drehzahlschwankungen ausgleichen, die durch bauartbedingte Unterschiede der Testbandkassetten 18, beispielsweise durch toleranzbehaftete Reibwiderstände der Spulen 26, 28 bedingt sein können.

Wie auch aus der Detailvergrößerung der Fig. 1 in axialer Draufsicht auf die Motorwelle 32 ersichtlich, umfasst die Regeleinrichtung 16 einen an dem Gleichstrommotor 30 angeordneten Drehgeber 38 zur Erfassung der IST-Drehzahl der Motorabtriebswelle 32. Dieser ist in dem gezeigten Ausführungsbeispiel als opto-elektronischer Encoder 40, speziell als auf der Motorabtriebswelle drehfest sitzendes Flügelrad 42 in Wirkverbindung mit einer das Flügelrad beidseitig umgreifenden, gerätefest angeordneten vierflügeligen Gabellichtschranke 44 ausgebildet. Das Flügelrad 42 kann auch an einer drehfest auf der Motorabtriebswelle sitzenden Getriebekomponente, beispielsweise dem Schneckenrad 34 angebracht bzw. angeformt sein.

Fig. 2 veranschaulicht den geschlossenen Regelkreis der Regeleinrichtung 16 in einem Blockschaltbild. Ein Vergleicher 46 ist zur Bildung einer Regeldifferenz e zwischen einer SOLL-Drehzahl $n_r$ bzw. SOLL-Kreisfrequenz $\omega_r$ (allgemein: $n = \omega/2\pi$) und der an der Motorwelle 32 mittels des Drehgebers 38 erfassten und über den Rückkopplungszweig 48 rückgespeisten IST-Kreisfrequenz $\omega$ ausgebildet. Ein eingangsseitig mit der Regeldifferenz e beaufschlagter Regelprozessor 50 sorgt für eine Reduzierung der Regeldifferenz. Zu diesem Zweck weist der Regelprozessor 50 ein Proportionalglied 52 und ein Integralglied 54 zweckmäßig in Form einer Software-Routine auf. Ausgangsseitig ist der Regelprozessor 50 mit einem Stellglied 56 gekoppelt, das als Pulsweitenmodulator den Gleichstrommotor 30 mit einer pulsweitenmodulierten Gleichspannung ansteuert. Der nachfolgende verzahnte Antriebsstrang 36 setzt die Antriebsrotation weitgehend abweichungsfrei in eine Translation des Testbandes 20 in der Bandkassette 18 um.

Fig. 3 veranschaulicht die genaue Bestimmung der IST-Drehzahl n aus dem Ausgangssignal 58 der Gabellichtschranke 44. Bei einer Umdrehung des vierflügeligen Flügelrads 42 in der Periodendauer T = 2π rad werden durch entsprechende Lichtschrankenunterbrechungen vier Impulse 60 erzeugt. Ein symbolisch dargestellter Taktgeber 62 zur Erzeugung von Zeittaktimpulsen 64 der Taktdauer d in Verbindung mit einem Digitalzähler 64 ermöglicht die Bestimmung der Impulszahl i zwischen zwei fallenden Flanken des Signals 58. Daraus ergibt sich die IST-Drehzahl n bzw. IST-Kreisfrequenz ω gemäß folgender Gleichung:

$$\omega \ (\mathrm{rad}/\mathrm{s}) = \frac{\frac{1}{4} * 2\pi}{i * d} \qquad (1)$$

Bei dieser Methode wird die Drehzahlbestimmung mit zunehmender Winkelgeschwindigkeit ungenauer. Bei einer den Anforderungen entsprechenden maximalen Winkelgeschwindigkeit von ω = 1128 rad/s wird entsprechend der Zählerauslegung eine Impulszahl i = 1392 mit einer Zählertoleranz von ±1 bestimmt. Daraus folgt eine minimale Genauigkeit der Drehzahlbestimmung von ±0.07%, was für die gewünschte Genauigkeit der Drehzahlregelung hinreichend ist. Bei einer dem Fachmann bekannten Auslegung der Regelglieder 52, 54 lässt sich somit eine Bandgeschwindigkeit des Testbandes 20 von 15 +/- 2 mm/s mit einer Einstellzeit im Bereich von 0,1 bis 0,25 s erreichen.

Durch einen Sollwertgenerator 66 (Fig. 2) der Regeleinrichtung 16 wird die SOLL-Drehzahl nach Maßgabe einer gleichbleibenden Bereitstellungszeit für die jeweilige Bereitstellung der analytischen Hilfsmittel 22 festgelegt. Zweckmäßig wird hierzu der die Bandgeschwindigkeit bestimmende und mit zunehmendem Hilfsmittelverbrauch erhöhte Bandwickeldurchmesser auf der Aufwickelspule 28 durch die aktuelle Testnummer i der fortlaufend nummerierten Testfelder 22 berücksichtigt. Die aktuelle Testnummer i wird vorteilhafterweise in einem auf der Testbandkassette 18 angebrachten Speichermittel, beispielsweise einem RFID-Chip 68 (Fig. 1) gespeichert und in den Sollwertgenerator 66 eingespeist. Dieser enthält einen Sollwertspeicher 70, in welchem der Testnummer i für die auf dem Testband 20 verteilten fünfzig Testfelder jeweils ein Wert für die einzuregelnde Winkelgeschwindigkeit ω gemäß folgender Tabelle zugeordnet ist:

Tabelle I

| i | ω (rad/s) | i | ω (rad/s) | i | ω (rad/s) | i | ω (rad/s) | i | ω (rad/s) |
|---|---|---|---|---|---|---|---|---|---|
| 1 | 985 | 11 | 883 | 21 | 799 | 31 | 731 | 41 | 672 |
| 2 | 974 | 12 | 874 | 22 | 792 | 32 | 724 | 42 | 667 |
| 3 | 964 | 13 | 865 | 23 | 785 | 33 | 718 | 43 | 662 |
| 4 | 953 | 14 | 856 | 24 | 778 | 34 | 712 | 44 | 657 |
| 5 | 942 | 15 | 848 | 25 | 770 | 35 | 706 | 45 | 652 |
| 6 | 931 | 16 | 839 | 26 | 763 | 36 | 700 | 46 | 647 |
| 7 | 922 | 17 | 831 | 27 | 757 | 37 | 694 | 47 | 642 |
| 8 | 912 | 18 | 822 | 28 | 750 | 38 | 689 | 48 | 637 |
| 9 | 902 | 19 | 815 | 29 | 743 | 39 | 684 | 49 | 632 |
| 10 | 892 | 20 | 807 | 30 | 737 | 40 | 678 | 50 | 627 |

Neben den Testfeldern 22 können auf den jeweiligen Abschnitten des Testbands 20 auch Funktionsfelder 72 vorgesehen sein, die eine Kalibrierung der photometrischen Messeinheit oder eine genaue Bandpositionierung für verschiedene Funktionen ermöglichen. Zur Bandpositionierung werden die Funktionsfelder 72 über einen von der photometrischen Messeinheit gesonderten optischen Bandsensor abgetastet, der nach Art einer Lichtschranke eine Photo-LED zur Beleuchtung und einen Photo-Transistor zur Erfassung der Reflexion umfasst. Damit wird beispielsweise beim Durchgang eines weißen Funktionsfeldes ein Rechtecksignal mit ansteigender und abfallender Signalflanke erfasst, wobei die Signalbreite von der Länge des Funktionsfeldes (in Bandlängsrichtung gesehen) und dessen Transportgeschwindigkeit abhängig. Für weitere Einzelheiten solcher Funktionsfelder und deren Verwendung wird auf die EP-A 2221608 Bezug genommen.

Zur Längenerfassung der unterschiedlichen Funktionsfelder kann die erfindungsgemäße Regeleinrichtung 16 und insbesondere der Drehgeber 38 vorteilhaft eingesetzt werden. Die Längenerfassung kann als zusätzliches Kontrollkriterium genutzt werden, beispielsweise auch um die aktuelle Position bei einer Gerätefehlfunktion wieder zu finden.

Im Falle einer definierten Bandgeschwindigkeitsregelung mittels der Regeleinrichtung kann die Funktionsfeldlänge auf besonders einfache Weise durch einen akkumulierten Zählwert von Zeittaktimpulsen 64 des Taktgebers 62 zwischen den oben erwähnten Signalflanken beim Durchgang des jeweiligen Funktionsfelds ermittelt werden.

Der Drehgeber 38 kann auch genutzt werden, um ein Maß L für die Funktionsfeldlänge selbst bei variierender Drehgeschwindigkeit direkt zu erfassen. Hierbei gilt

$$L = u * 2\pi R \qquad (2),$$

wobei u die Umdrehungen der Aufwickelspule 28 und R deren Bandwickeldurchmesser bezeichnen. Da das Verhältnis aus Motordrehzahl n und Spulendrehzahl u durch das Getriebeübersetzungsverhältnis v bestimmt ist, ergibt sich aus der Zahl Pi der Impulse 60 des Drehgebers zwischen den Signalflanken beim Durchgang des jeweiligen Funktionsfelds

$$L = Pi * 2\pi R / 4v \qquad (3).$$

Wenn also die Impulszahl Pi der Gabellichtschranke 44 direkt erfasst wird und der Bandwickeldurchmesser anhand der Testnummer bekannt ist, kann die Länge L des betreffenden Funktionsfeldes unmittelbar abgeleitet werden.

## Patentansprüche

1. Analytisches Handgerät, insbesondere für Blutzuckertests, mit einer austauschbaren Testbandkassette (18), die ein mit einer Vielzahl von analytischen Hilfsmitteln (22) versehenes Testband (20) enthält, einem einen Gleichstrommotor (30) und ein mit der Testbandkassette (18) koppelbares Getriebe (34,36) aufweisenden Bandantrieb (14) zum sukzessiven Bereitstellen der analytischen Hilfsmittel (22), und einer Regeleinrichtung (16) zur Drehzahlregelung des Gleichstrommotors (30), **dadurch gekennzeichnet, dass** die Regeleinrichtung (16) einen an dem Gleichstrommotor (30) angeordneten Drehgeber (38) zur Erfassung der IST-Drehzahl der Motorabtriebswelle (32) aufweist.

2. Handgerät nach Anspruch 1, **dadurch gekennzeichnet, dass** der Drehgeber (38) einen die Drehung der Motorabtriebswelle (32) optisch abtastenden und dabei eine zur Drehzahl proportionale Rate von elektrischen Impulsen als Ausgangssignal erzeugenden opto-elektronischen Encoder (40) aufweist.

3. Handgerät nach Anspruch 1 oder 2, **dadurch gekennzeichnet, dass** der Drehgeber (38) einen drehfest auf der Motorabtriebswelle (32) sitzenden Unterbrecher (42), insbesondere ein Flügelrad oder eine Lochscheibe und eine mit dem Unterbrecher (42) zusammenwirkende gerätefeste Lichtschranke (44), vorzugsweise eine Gabellichtschranke aufweist.

4. Handgerät nach einem der Ansprüche 1 bis 3, **gekennzeichnet durch** einen zur Erzeugung von Zeittaktimpulsen ausgebildeten Taktgeber (62) und einen Zähler (64) zum Zählen von Zeittaktimpulsen zwischen zwei Signalflanken eines Ausgangssignals (58) des Drehgebers (38).

5. Handgerät nach einem der Ansprüche 1 bis 4, **dadurch gekennzeichnet, dass** die Regeleinrichtung (16) einen Vergleicher (46) zur Bildung einer Regeldifferenz zwischen der IST-Drehzahl und einer als Führungsgröße vorgegebenen SOLL-Drehzahl aufweist.

6. Handgerät nach einem der Ansprüche 1 bis 5, **dadurch gekennzeichnet, dass** die Regeleinrichtung (16) einen eingangsseitig mit einer Regeldifferenz beaufschlagbaren Regelprozessor (50) zur Einstellung einer Soll-Drehzahl in einem geschlossenen Regelkreis aufweist.

7. Handgerät nach einem der Ansprüche 1 bis 6, **dadurch gekennzeichnet, dass** der Regelprozessor (50) vorzugsweise durch eine Software-Routine gebildete Proportional- und Integral-Regelglieder (52,54) aufweist.

8. Handgerät nach einem der Ansprüche 1 bis 7, **dadurch gekennzeichnet, dass** die Regeleinrichtung (16) einen Pulsweitenmodular als Stellglied (56) zur Ansteuerung des Gleichstrommotors (30) mit einer pulsweitenmodulierten Gleichspannung aufweist.

9. Handgerät nach einem der Ansprüche 1 bis 8, **dadurch gekennzeichnet, dass** die Regeleinrichtung (16) dazu eingerichtet ist, eine Bandgeschwindigkeit des Testbandes (20) von 15 +/- 2 mm/s mit einer Einstellzeit im Bereich von 0,1 bis 0,25 s zu erreichen.

10. Handgerät nach einem der Ansprüche 1 bis 9, **dadurch gekennzeichnet, dass** die Regeleinrichtung (16) einen Sollwertgenerator (66) zur Festlegung einer aktuellen SOLL-Drehzahl nach Maßgabe einer gleichbleibenden Bereitstellungszeit für die jeweilige Bereitstellung der analytischen Hilfsmittel (22) aufweist.

11. Handgerät nach einem der Ansprüche 1 bis 10, **gekennzeichnet durch** einen Sollwertspeicher (70) zur Speicherung einer Sollwerttabelle, in welcher einer Testnummer der fortlaufend nummerierten analytischen Hilfsmittel jeweils ein Wert für die SOLL-Drehzahl des Gleichstrommotors (30) zugeordnet ist.

12. Handgerät nach einem der Ansprüche 1 bis 11, **dadurch gekennzeichnet, dass** die Testbandkassette (18) mit einem insbesondere als RFID-Chip ausgebildeten Speichermittel (68) zur verbrauchsabhängigen Speicherung einer Testnummer des aktuell bereitzustellenden analytischen Hilfsmittels (22) versehen ist.

13. Handgerät nach einem der Ansprüche 1 bis 12, **dadurch gekennzeichnet, dass** die analytischen Hilfsmittel (22) durch Bandtransport von einer gegenüber der Umgebung abgeschirmten Vorratsspule (26) abziehbar und an einer Applikationsstelle (24) bereitstellbar sind.

14. Verfahren zum Betrieb eines analytischen Handgeräts insbesondere nach einem der vorhergehenden Ansprüche, bei welchem ein mit einer Vielzahl von analytischen Hilfsmitteln (22) versehenes Testband (20) in Form einer austauschbaren Testbandkassette (18) in dem Handgerät (10) eingesetzt wird, wobei die analytischen Hilfsmittel (22) durch einen Bandantrieb (14) sukzessive bereitgestellt werden und dabei die Drehzahl eines Gleichstrommotors (30) des Bandantriebs (14) mittels einer Regeleinrichtung (16) geregelt wird, **dadurch gekennzeichnet, dass** die IST-Drehzahl der Motorabtriebswelle (32) durch einen an dem Gleichstrommotor (30) angeordneten Drehgeber (38) der Regeleinrichtung (16) erfasst wird.

## Claims

1. A hand-held analytical device, in particular for blood sugar tests, comprising a replaceable test tape cassette (18) which contains a test tape (20) provided with a plurality of analytical aids (22), a tape drive (14) comprising a DC motor (30) and gearing (34, 36) which can be coupled to the test tape cassette (18) for successive provision of the analytical aids (22) and a control device (16) for rotational speed control of the DC motor (30), **characterized in that** the control device (16) comprises a rotary pickup (38) arranged on the DC motor (30) in order to record the ACTUAL rotational speed of the motor drive shaft (32).

2. The hand-held device as claimed in claim 1, **characterized in that** the rotary pickup (38) comprises an optoelectronic encoder (40) optically sampling the rotation of the motor drive shaft (32) while generating a rate of electrical pulses proportional to the rotational speed as an output signal.

3. The hand-held device as claimed in claim 1 or 2, **characterized in that** the rotary pickup (38) comprises an interrupter (42), in particular a vaned wheel or a perforated disk, seated in a rotationally fixed manner on the motor drive shaft (32) and a light barrier (44) fixed to the device and interacting with the interrupter (42), preferably a fork light barrier.

4. The hand-held device as claimed in one of claims 1 to 3, **characterized by** a clock generator (62) formed in order to generate time clock pulses and a counter (64) for counting time clock pulses between two signal edges of an output signal (58) of the rotary pickup (38).

5. The hand-held device as claimed in one of claims 1 to 4, **characterized in that** the control device (16) comprises a comparator (46) for forming a control difference between the ACTUAL rotational speed and a SETPOINT rotational speed specified as a control variable.

6. The hand-held device as claimed in one of claims 1 to 5, **characterized in that** the control device (16) comprises a control processor (50), to which a control difference can be applied on the input side, for adjusting a setpoint rotational speed in a closed control loop.

**7.** The hand-held device as claimed in one of claims 1 to 6, **characterized in that** the control processor (50) preferably comprises proportional and integral control elements (52, 54) formed by a software routine.

**8.** The hand-held device as claimed in one of claims 1 to 7, **characterized in that** the control device (16) comprises a pulse-width modulator as an actuating element (56) for driving the DC motor (30) with a pulse-width modulated DC voltage.

**9.** The hand-held device as claimed in one of claims 1 to 8, **characterized in that** the control device (16) is adapted in order to achieve a tape speed of the test tape (20) of $15 \pm 2$ mm/s with an adjustment time in the range of from 0.1 to 0.25 s.

**10.** The hand-held device as claimed in one of claims 1 to 9, **characterized in that** the control device (16) comprises a setpoint value generator (66) for establishing a current SETPOINT rotational speed in accordance with a constant provision time for the respective provision of the analytical aids (22).

**11.** The hand-held device as claimed in one of claims 1 to 10, **characterized by** a setpoint value memory (70) for storing a setpoint value table, in which a value of the SETPOINT rotational speed of the DC motor (30) is respectively assigned to a test number of the continuously numbered analytical aids.

**12.** The hand-held device as claimed in one of claims 1 to 11, **characterized in that** the test tape cassette (18) is provided with a storage means (68), formed in particular as an RFID chip, for usage-dependent storage of a test number of the analytical aid (22) currently to be provided.

**13.** The hand-held device as claimed in one of claims 1 to 12, **characterized in that** the analytical aids (22) can be drawn by tape transport from a stock spool (26) shielded from the surroundings and can be provided at an application site (24).

**14.** A method for operating a hand-held analytical device, in particular as claimed in one of the preceding claims, in which a test tape (20) provided with a plurality of analytical aids (22) is used in the form of a replaceable test tape cassette (18) in the hand-held device, the analytical aids (22) being provided successively by a tape drive (14) and the rotational speed of a DC motor (30) of the tape drive (14) being controlled by means of a control device (16), **characterized in that** the ACTUAL rotational speed of the motor drive shaft (32) is recorded by a rotary pickup (38) of the control device (16), which pickup is arranged on the DC motor (30).

**Revendications**

**1.** Appareil manuel d'analyse, notamment pour des tests de glycémie, comprenant une cassette de bandelette de test (18) interchangeable qui contient une bandelette de test (20) pourvue d'une pluralité de moyens analytiques (22), un entraînement de bandelette (14) équipé d'un moteur à courant continu (30) et d'un mécanisme à engrenages (34, 36) pouvant être accouplé avec la cassette de bandelette de test (18) pour mettre successivement à disposition les moyens analytiques (22), et un dispositif de régulation (16) pour réguler la fréquence de rotation du moteur à courant continu (30), **caractérisé en ce que** le dispositif de régulation (16) comprend un résolveur (38) situé sur le moteur à courant continu (30), destiné à saisir la fréquence de rotation réelle de l'arbre mené (32) du moteur.

**2.** Appareil manuel selon la revendication 1, **caractérisé en ce que** le résolveur (38) comprend un codeur (40) optoélectronique qui balaye optiquement la rotation de l'arbre mené (32) du moteur et génère, en tant que signal de sortie, un taux d'impulsions électriques proportionnel à la fréquence de rotation.

**3.** Appareil manuel selon la revendication 1 ou 2, **caractérisé en ce que** le résolveur (38) comprend un rupteur (42), notamment une roue à ailettes ou un disque perforé, monté de manière fixe en rotation sur l'arbre mené (32) du moteur et une barrière lumineuse (44), de préférence une barrière lumineuse fourchue, fixe sur l'appareil et coopérant avec le rupteur (42).

**4.** Appareil manuel selon l'une des revendications 1 à 3, **caractérisé par** une horloge (62) conçue pour générer des impulsions d'horloge et un compteur (64) pour compter les impulsions d'horloge entre deux flancs de signal d'un signal de sortie (58) du résolveur (38).

**5.** Appareil manuel selon l'une des revendications 1 à 4, **caractérisé en ce que** le dispositif de régulation (16) comprend un comparateur (46) destiné à former une variable de différence de régulation entre la fréquence de rotation réelle et une fréquence de rotation de consigne prédéterminée en tant que grandeur de référence.

**6.** Appareil manuel selon l'une des revendications 1 à 5, **caractérisé en ce que** le dispositif de régulation (16) comprend un processeur de régulation (50), auquel une variable de différence de régulation peut être appliquée côté entrée, destiné à régler une fréquence de rotation de consigne dans une boucle d'asservissement fermée.

**7.** Appareil manuel selon l'une des revendications 1 à 6, **caractérisé en ce que** le processeur de régulation (50) comprend des éléments régulateurs proportionnels intégraux formés de préférence par une routine logicielle.

**8.** Appareil manuel selon l'une des revendications 1 à 7, **caractérisé en ce que** le dispositif de régulation (16) comprend un modulateur d'impulsions en largeur en tant que composant de réglage (56) destiné à commander le moteur à courant continu (30) avec une tension continue modulée en largeur d'impulsion.

**9.** Appareil manuel selon l'une des revendications 1 à 8, **caractérisé en ce que** le dispositif de régulation (16) est conçu pour atteindre une vitesse de bandelette de la bandelette de test (20) de $15\pm2$ mm/s en un temps de réglage de l'ordre de 0,1 s à 0,25 s.

**10.** Appareil manuel selon l'une des revendications 1 à 9, **caractérisé en ce que** le dispositif de régulation (16) comprend un générateur de valeur de consigne (66) destiné à fixer une fréquence de rotation de consigne effective en fonction d'un temps de mise à disposition constant pour la mise à disposition respective des moyens analytiques (22).

**11.** Appareil manuel selon l'une des revendications 1 à 10, **caractérisé par** une mémoire de valeurs de consigne (70) destinée au stockage d'une table de valeurs de consigne dans laquelle une valeur de la fréquence de rotation de consigne du moteur à courant continu (30) est associée, à chaque fois, à un numéro de test des moyens analytiques numérotés de manière continue.

**12.** Appareil manuel selon l'une des revendications 1 à 11, **caractérisé en ce que** la cassette de bandelette de test (18) est pourvue d'un moyen de stockage (68) exécuté, notamment, en tant que puce RFID, destiné au stockage, en fonction de la consommation, d'un numéro de test du moyen analytique (22) devant effectivement être mis à disposition.

**13.** Appareil manuel selon l'une des revendications 1 à 12, **caractérisé en ce que** les moyens analytiques (22) peuvent, grâce au transport de la bandelette, être dévidés d'une bobine de réserve (26) protégée contre l'environnement et mis à disposition au niveau d'un point d'application (24).

**14.** Procédé de fonctionnement d'un appareil manuel d'analyse, notamment selon l'une des revendications précédentes, dans lequel une bandelette de test (20) pourvue d'une pluralité de moyens analytiques (22) est insérée, sous forme d'une cassette de bandelette de test (18) interchangeable, dans l'appareil manuel (10), les moyens analytiques (22) étant mis à disposition successivement grâce à un entraînement de bandelette (14) et la fréquence de rotation d'un moteur à courant continu (30) de l'entraînement de bandelette (14) étant régulée au moyen d'un dispositif de régulation (16), **caractérisé en ce que** la fréquence de rotation réelle de l'arbre mené (32) du moteur est saisie par un résolveur (38) du dispositif de régulation (38) situé sur le moteur à courant continu (30).

Fig.1

Fig.2

Fig.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2177155 A1
- EP 2221608 A